# EUROPÄISCHE PATENTSCHRIFT

(11) **EP 2 066 230 B1**
(45) Veröffentlichungstag und Bekanntmachung des Hinweises auf die Patenterteilung: **28.04.2010**
(21) Anmeldenummer: 08715993.5
(22) Anmeldetag: 22.02.2008
(51) Int. Cl.: A61B 5/00, A61B 5/22

(54) **VORRICHTUNG ZUR INDIREKTEN MESSUNG OKKLUSALER KRÄFTE**
DEVICE FOR INDIRECTLY MEASURING OCCLUSAL FORCES
DISPOSITIF DE MESURE INDIRECTE DE FORCES OCCLUSALES

(30) Priorität: 28.02.2007 DE 102007009910
(43) Veröffentlichungstag der Anmeldung: 10.06.2009
(73) Patentinhaber: Sense Inside GmbH, 80335 München (DE)
(72) Erfinder: SCHOLZ, Alexander, 84558 Kirchweihdach (DE); CLAUSS, Johannes, 80801 München (DE)
(74) Vertreter: Müller - Hoffmann & Partner
(86) Internationale Anmeldenummer: PCT/EP2008/001442
(87) Internationale Veröffentlichungsnummer: WO 2008/104339

(56) Entgegenhaltungen:
- DE-A1-102004 043 665
- US-A- 5 078 153
- US-A- 5 846 211
- US-A- 6 089 864
- BLAMPHIN C N J ET AL: "A SIMPLE INSTRUMENT FOR THE MEASUREMENT OF MAXIMUM OCCLUSAL FORCE IN HUMAN DENTITION" PROCEEDINGS OF THE INSTITUTION OF MECHANICAL ENGINEERS.JOURNAL OF ENGINEERING IN MEDICINE. PART H, MECHANICAL ENGINEERING PUBLICATIONS LTD, LONDON, GB, Bd. 204, Nr. H02 PART H, 1. Januar 1990 (1990-01-01), Seiten 129-131, XP000175116 ISSN: 0954-4119

## Beschreibung

Die Erfindung betrifft eine Vorrichtung, insbesondere eine Zahnschiene, mit wenigstens einem Sensor, zur Messung von okklusalen Kräften bei einem Schlussbiss von Zähnen. Die Erfindung betrifft zudem ein System zur Diagnose oder Therapie von Bruxismus. Die Erfindung betrifft weiterhin Verfahren zum Bestimmen der Position oder der Größe von okklusalen Kräften auf die Vorrichtung.

Im Rahmen verschiedener diagnostischer oder therapeutischer Verfahren in der Medizin bzw. Zahnmedizin werden bereits Zahnschienen eingesetzt, die an einem oder mehreren Zähnen angebracht werden können und verschiedene Aufgaben übernehmen sollen. Insbesondere bei der Behandlung von Bruxismus (umgangssprachlich auch "Zähneknirschen" oder "Zähnepressen") werden verschiedene Zahnschienen eingesetzt.

Ein Bruxismuspatient vollzieht ein parafunktionales Aufeinanderpressen der Zähne, das in der Regel unbewusst geschieht. Bei den am Tag und in der Nacht auftretenden Bruxismusereignissen wird häufig mit starker Kraft eine Okklusion der Zähne herbeigeführt, also ein Schlussbiss der oberen und unteren Zähne. Dadurch entstehen neben störenden Auswirkungen wie Zähneknirschen auch schwerwiegende direkte und indirekte pathologische Folgen für den Patienten, wie etwa Zahnverschleiß, Überlastung des Zahnhalteapparates, des Kiefergelenkes sowie der Kaumuskulatur, Tinnitus oder Schwindel.

Für die Behandlung von Bruxismus wurden neben rein mechanischen Zahnschienen zum Schonen der Zähne vor Verschleiß auch elektronische Zahnschienen entwickelt, in welchen Sensoren zur Messung des Aufbissdrucks angeordnet sind. Hierbei können Messwerte per Kabel an eine entsprechende Empfangseinheit ausgegeben werden, mit deren Hilfe eine diagnostische Datenauswertung erfolgt. Auch eine Weitergabe von durch eine in der Zahnschiene angeordnete Elektronik aufgenommenen Daten drahtlos über eine Telemetriestrecke an eine Empfangseinheit wurde bereits realisiert.

Bei Bruxismusereignissen oder bei anderen Parafunktionen der Zähne bzw. der Kiefermuskulatur auftretende Kräfte auf die Zähne können folglich erfasst und analysiert werden. So zeigt beispielsweise US-Patent Nr. 5,078,153 eine Zahnschiene, bei der sich ein piezoelektrischer Film als Sensor zwischen den Kauflächen der Backenzähne befindet, um so einen Aufbissdruck an den Kauflächen der Zähne zu erfassen. In US-Patent Nr. 5,586,562 ist eine Vorrichtung zum Erfassen von Aufbissdruck gezeigt, bei der ein elektrischer Sensor über der Kaufläche eines Zahnes angeordnet ist, um dort Informationen über einen Aufbissdruck zu erfassen.

Den bestehenden elektronischen Zahnschienen zur Diagnose oder Therapie von Bruxismus ist gemeinsam, dass ein Aufbissdruck stets durch einen elektrischen Sensor gemessen wird, der im Okklusionsbereich der Zähne angeordnet ist, also in dem Bereich, in dem gegenüberliegende Zähne des Oberkiefers und des Unterkiefers sich in einer Schlussbissstellung berühren.

Im Folgenden sei unter einer Okklusionsregion jene Fläche der Zahnschiene verstanden, die bei sachgerechter Anwendung der Schiene bei einem Aufbiss der Zähne 11 bis 18 bzw. 21 bis 28 (nach Federation Dentaire International, FDI) des Oberkiefers oder der Zähne 31 bis 38 bzw. 41 bis 48 des Unterkiefers (im Folgenden auch "obere" und "untere" Zähne) mit einem Zahn in Berührung kommen, im Wesentlichen also die die Kauflächen der Zähne antagonisierenden Bereiche der Schiene.

Aus US 5,846,211 A ist eine Messvorrichtung für okklusale Kräfte bekannt, bei der eine Okklusionskraft über einen in einem Okklusionsbereich von Zähnen angeordneten Kraftübertragungsbereich aufgenommen und an einen Druckdetektor weitergegeben wird. Ein Griff zum Festhalten der Vorrichtung ist so ausgebildet, dass er bei einem Aufbiss eines Probanden außerhalb seines Mundraumes angeordnet ist und festgehalten werden kann. Der Aufbiss erfolgt direkt auf eine Druckaufnahmescheibe und den Kraftübertragungsbereich.

XP 000175116. Blamphin C. N. J; Branfield T. R.: Jobbins B: Fisher J; Watson C. J; Redfen E. J., A simple instrument for the measurement of maximum occlusal force in human dentition". Proceedings of the Institution of Mechanical Engineers, Part H (Journal of Engineering in Medicine), 1990. UK. ISSN 0954-4119, vil. 204. nr. H2, pg. 129-131 offenbart eine zahnmedizinische Vorrichtung zum Messen einer maximalen Okklusionskraft. Ein Schlussbiss erfolgt auf Polymerbereiche eines mit einem Scharnier zusammengehaltenen zweigliedrigen Rahmens. Zwischen den beiden Gliedern des Rahmens ist ein Kraftübertrager angeordnet, so dass dieser eine über den Rahmen weitergeleitete Kraft aufnehmen kann.

Der Erfindung liegt die Aufgabe zugrunde, eine Vorrichtung zur Messung okklusaler Kräfte anzugeben, sowie ein Verfahren zum Auswerten von Messgrößen.

Die Aufgabe wird erfindungsgemäß durch eine Vorrichtung gemäß Anspruch 1 gelöst. Ein zugeordnetes Verfahren ergibt sich aus Anspruch 12. Weiterentwicklungen der Erfindung sind den abhängigen Ansprüchen definiert.

Bei einer erfindungsgemäßen Vorrichtung zum Messen okklusaler Kräfte bei einem Schlussbiss von Zähnen ist wenigstens ein Sensor vorgesehen, der außerhalb eines Okklusionsbereiches der Zähne angeordnet ist. Unter okklusalen Kräften werden hier alle Kräfte verstanden, die bei einer auch "Schlussbiss" oder "Interkuspidation" genannten Okklusion zwischen Zähnen des Oberkiefers und des Unterkiefers entstehen können. Diese Kräfte können entsprechend der Bewegungsrichtung der Zähne beim Schlussbiss verschiedene Richtungen besitzen. Eine Okklusion kann beispielsweise eine statische Okklusion, eine habituelle Okklusion, eine zentrische Okklusion oder eine maximale Interkuspidation sein und ist ferner nicht auf eine dieser oder weiterer möglicher Okklusionsformen beschränkt, sondern kann auch eine Mischform aus verschiedenen Okklusionsformen sein.

Unter einem Okklusionsbereich der Zähne wird der Bereich zwischen oberen und unteren Zähnen bei einem Schussbiss verstanden, der zwischen den Okklusionspunkten der oberen und unteren Zähne liegt, also zwischen jenen Stellen, an denen sich bei einem Schlussbiss die oberen und unteren Zähne berühren. Somit ist der Okklusionsbereich der Zähne im Wesentlichen der Bereich, der zwischen den Kauflächen der oberen und unteren Zähne liegt. Jedoch umfasst der Okklusionsbereich auch solche Bereiche zwischen den oberen und unteren Zähnen, bei dem sich Okklusionspunkte von Zähnen des Oberkiefers und des Unterkiefers gegenüberliegen, die aufgrund einer beliebigen Okklusionsform auftreten. Der Okklusionsbereich kann dementsprechend auch als Projektionsbereich der Kauflächen der oberen und unteren Zähne aufeinander verstanden werden.

Der vorgesehene Sensor ist somit nicht zwischen den Kauflächen der oberen und unteren Zähne, sondern außerhalb des Bereiches zwischen den Kauflächen, also des Okklusionsbereiches der Zähne angeordnet. Ein Sensor kann beispielsweise seitlich der Backenzähne oder vor oder hinter den Schneidezähnen angeordnet sein. Anders ausgedrückt ist der Sensor außerhalb eines Schnittbereiches zwischen der Okklusionsebene (definiert als Fläche durch die Verbindungslinien zwischen dem Berührungspunkt der Schneidekanten der unteren mittleren Schneidezähne 31 und 41 und dem sog. "distalen Höcker" der Zähne 36 und 46 durch die Lippenschlusslinie) und dem Okklusionsbereich der Zähne angeordnet. Entsprechend ist bei der Anordnung mehrerer Sensoren jeder dieser Sensoren außerhalb der Kauflächen und des Bereiches zwischen den Kauflächen angeordnet.

Bei einer Ausführungsform kann ein Sensor an der Außenseite eines Zahnes, also beispielsweise am Zahnschmelz eines Zahnes oder an seinem Zahnzement anordenbar sein, oder auch an der Außenseite einer Zahnfüllung, einer Plombe, einer Zahnkrone oder eines Zahn-Inlays. Der Sensor kann ebenso an der Außenseite eines Zahnimplantates, eines Kunstzahnes oder einer Zahnprothese anordenbar sein. In den vorangehenden Fällen spielt es keine Rolle, ob der Sensor an der von der Zunge weggerichteten, äußeren Seite der Zähne, oder zur Zunge hingerichteten, inneren Seite der Zähne anordenbar ist, solange der Sensor entsprechend obiger Darstellung nicht im Okklusionsbereich der Zähne angeordnet ist.

Bei einer weiteren Ausführungsform kann der Sensor auch innerhalb eines Zahnes, also beispielsweise innerhalb seines Zahnschmelzes oder seines Zahnzements anordenbar sein, oder auch innerhalb einer Zahnfüllung, einer Plombe, einer Zahnkrone oder eines Zahn-Inlays. Der Sensor kann ebenso innerhalb eines Zahnimplantates, eines Kunstzahnes oder einer Zahnprothese anordenbar sein.

Bei der erfindungsgemäßen Vorrichtung ist ein Träger vorgesehen, der eine Okklusionsregion aufweist. Hierbei wird unter einer Okklusionsregion ein zusammenhängender oder nicht zusammenhängender Bereich des Trägers verstanden, der zum Einen die Okklusionsbereiche der oberen Zähne mit dem Träger und der unteren Zähne mit dem Träger umfasst. Zum Anderen umfasst die Okklusionsregion des Trägers mindestens einen Bereich zwischen den Okklusionsbereichen der oberen bzw. unteren Zähne mit dem Träger. Entsprechend den obigen Beschreibungen liegt eine Okklusionsregion des Trägers zwischen den Kauflächen der oberen und unteren Zähne bzw. innerhalb eines Okklusionsgebietes der Zähne. Eine derartige Definition der Okklusionsbereiche und Okklusionsregionen ist notwendig, da unterschieden werden kann zwischen einer Okklusion von Zähnen, also der Okklusion im eigentlichen medizinischen Sinne, und einer Okklusion von Zähnen mit einem dazwischen liegendem Träger.

Im letztgenannten Fall besteht kein direkter Antagonistenkontakt zwischen oberen und unteren Zähnen, da der Träger einen Berührungskontakt verhindert. Jedoch entsteht eine Okklusion, also ein Schlussbisskontakt, zwischen oberen Zähnen mit dem Träger sowie eine Okklusion zwischen unteren Zähnen mit dem Träger. Diese oberen und unteren Okklusionsbereiche des Trägers und die Bereiche des Trägers, die zwischen diesen Okklusionsbereichen liegen, definieren die Oklusionsregion des Trägers.

Entsprechend der Anordnung nach den oben beschriebenen Ausführungsformen ist ein Sensor der Vorrichtung außerhalb der Okklusionsregion des Trägers angeordnet, also außerhalb eines Gebietes des Trägers, bei dem sich die Kauflächen der oberen und unteren Zähne bei einem Schlussbiss gegenüberliegen.

In einer Ausführungsform der Erfindung ist die Okklusionsregion des Trägers im Wesentlichen parallel zu einer Kaufläche der Zähne anordenbar. Mindestens eine Kaufläche der oberen oder der unteren Zähne kann somit von dem Träger bedeckt sein. Selbstverständlich soll bei einem Schlussbiss zwischen Oberkiefer und Unterkiefer sowohl eine Kaufläche der oberen Zähne als auch eine Kaufläche der unteren Zähne von dem Träger bedeckt sein, so dass eine Okklusion zwischen oberen und unteren Zähnen mit dem Träger stattfinden kann, selbst wenn der Träger nur auf Zähnen des oberen oder des unteren Kiefers aufgeklemmt ist. Zudem besitzt der Träger bei dieser Ausführungsform der Erfindung eine Flankenregion, die im Wesentlichen senkrecht zu einer Kaufläche der oberen oder unteren Zähne anordenbar ist. Eine Flankenregion ist demnach eine Region des Trägers, die an den Seiten der Backen-, Eck- oder Schneidezähne und außerhalb eines Okklusionsbereiches der Zähne liegt.

Der Träger kann eine Kunststoffschiene sein, wie sie auch als "Zahnschiene" bezeichnet wird, die auf einem Zahn oder mehreren Zähnen aufklemmbar ist. Dabei kann die Schiene auf mindestens einen Zahn des Oberkiefers oder des Unterkiefers aufgeklemmt, aufgesteckt, aufgeschoben, oder auf eine andere Weise lösbar oder unlösbar befestigt werden. Möglich ist auch, dass die Kunststoffschiene sowohl auf mindestens einen Zahn des Oberkiefers als auch auf mindestens einen Zahn des Unterkiefers befestigbar ist. Das Material der Kunststoffschiene kann insbesondere ein zahnmedizinischer Kunststoff sein, der ungiftig und abriebfest ist. Vorteilhaft ist darüber hinaus ein Kunststoff, der im ausgehärteten Zustand weitgehend durchsichtig ist. Es können aber auch beliebige andere Kunststoffe oder auch weitere Materialien verwendet werden.

Bei einer Ausführungsform der vorliegenden Erfindung ist ein Sensor vollständig innerhalb des Trägers angeordnet, so dass der Sensor vollständig von dem Träger umschlossen ist und nicht aus diesem hervorsteht oder an einer Außenseite des Trägers liegt.

Möglich ist auch die Anordnung eines Sensors oder mehrerer Sensoren innerhalb der Flankenregion des Trägers, beispielsweise in dem Bereich des Trägers hinter einem Schneidezahn, oder seitlich eines Backenzahnes.

Ein Sensor erfasst eine physikalischen Kraft durch das Sensorisieren von durch die Wirkung der Kraft entstehenden skalaren Größen. Somit kann ein Sensor ein Dehnungssensor zum Sensorisieren einer Dehnung, ein Biegungssensor zum Sensorisieren einer Biegung, ein Vibrationssensor zum Sensorisieren einer Vibration, ein Spannungssensor zum Sensorisieren einer mechanischen Spannung oder Verspannung, ein Verschiebungssensor zum Sensorisieren einer mechanischen Verschiebung, oder ein Drucksensor zum Sensorisieren eines Druckes sein. Jedoch ist selbstverständlich auch die Verwendung eines Sensors zum direkten Sensorisieren gerichteter physikalischer Größen, wie Kraftsensoren zum sensorisieren von Betrag und Richtung einer Kraft, möglich.

In einer Ausführungsform der erfindungsgemäßen Vorrichtung ist ein Sensor ein piezoelektrischer Sensor zum Umwandeln mechanischen Druckes in elektrische Spannung. Ein Sensor kann erfindungsgemäß auch ein Dehnungsmessstreifen zum Umwandeln mechanischer Biegung in die Änderung eines ohmschen Widerstandes sein.

In einer weiteren Ausführungsform der Erfindung sind mehrere Sensoren vorgesehen, welche die gleiche Funktion oder auch unterschiedliche Funktionen besitzen können, also beispielsweise unterschiedliche physikalische Größen sensorisieren können oder unterschiedlich aufgebaut sind. So können auch unterschiedlich aufgebaute Sensoren zum Sensorisieren der gleichen Größen nach dem gleichen Messprinzip oder auch nach unterschiedlichen Messprinzipien verwendet werden.

Bei einer Ausführungsform der Erfindung ist eine elektronische Einheit vorgesehen, die durch einen Sensor erfasste Messdaten aufnehmen oder auswerten kann. Hierfür ist die elektronische Einheit vorzugsweise mit einem Sensor leitend verbunden. Möglich ist jedoch auch eine drahtlose Verbindung zwischen einem Sensor und der elektronischen Einheit. Die elektronische Einheit kann in einer Flankenregion des Trägers seitlich von Backenzähnen angeordnet sein. Weiterhin kann die elektronische Einheit auch eine Speichereinheit umfassen, zum Speichern von Messgrößen.

Bei einer weiteren Ausführungsform der vorliegenden Erfindung umfasst die Vorrichtung eine Sendeeinheit zum Senden von Messgrößen, die durch einen Sensor erfasst wurden oder erfasst werden oder die von der elektronischen Einheit aufgenommen oder ausgewertet wurden oder werden.

Der Träger kann mindestens zwei unterschiedliche Bereiche mit unterschiedlichen physikalischen bzw. mechanischen Eigenschaften wie Härte, Steifigkeit oder Festigkeit aufweisen. Der Träger kann beispielsweise aus zwei oder mehreren unterschiedlichen Arten von Kunststoff bestehen, die eine unterschiedliche Härte besitzen.

Mindestens zwei Sensoren können vollständig innerhalb jeweils eines der unterschiedlichen Bereiche mit unterschiedlichen mechanischen Eigenschaften angeordnet sein, so dass beispielsweise ein Sensor innerhalb eines ersten Bereiches des Trägers mit einer ersten Härte und ein zweiter Sensor innerhalb eines Bereiches des Trägers mit einer zweiten Härte angeordnet ist. Durch diese Anordnung können Vergleiche zwischen den Wirkungen von Kräften auf die unterschiedlichen Bereiche gezogen werden, die für die Auswertung der Messdaten und insbesondere für das Lokalisieren und das quantitative Bestimmen etwa eines Druckes verwendet werden können. Zu diesem Zwecke können mindestens zwei Sensoren erfindungsgemäß auch an mindestens zwei unterschiedlichen Bereichen mit unterschiedlichen physikalischen bzw. mechanischen Eigenschaften angrenzend angeordnet sein, so dass sie nicht vollständig innerhalb der unterschiedlichen Bereiche liegen.

Es können die Position und die Größe von okklusalen Kräften bestimmt werden, obwohl ein Sensor nicht in einem Okklusionsbereich der Zähne bzw. einer Okklusionsregion des Trägers angeordnet sind. Bei dem entsprechenden erfindungsgemäßen Verfahren werden mindestens zwei Sensoren innerhalb eines Trägers, jedoch außerhalb einer Okklusionsregion des Trägers bereitgestellt. Vorzugsweise sind die Sensoren entlang der Okklusionsregion des Trägers so verteilt, dass Kräfte über die gesamte Okklusionsregion erfasst werden können, und somit keine Bereiche in der Okklusionsregion auftreten, in denen Kräfte von keinem der bereitgestellten Sensoren erfasst werden können. Beim Auftreten eines Bruxismusereignisses, also beispielsweise eines Druckes auf einen Punkt in der Okklusionsregion erfassen die Sensoren die auftretenden Drücke unabhängig voneinander. Bei drei bereitgestellten Sensoren erhält man dadurch drei Intensitätswerte des Druckes. Ein quantitatives Bestimmen des auftretenden Okklusionsdruckes erfolgt durch einen Vergleich der Amplituden, also der Intensitätswerte der einzelnen Sensoren. Ein Lokalisieren des auftretenden Bruxismusereignisses erfolgt ebenfalls durch einen Vergleich der Amplituden, also der Intensitätswerte der einzelnen Sensoren. Je höher die Amplituden der Messwerte an einem Sensor sind, desto stärker ist der zugrunde liegende Druck bzw. desto näher liegt eine Krafteinwirkung an dem entsprechenden Sensor. Umgekehrt registriert ein Sensor die Folgen einer schwachen Krafteinwirkung bzw. eine weit entfernte Einwirkung als schwächeres Messsignal. Bei Kenntnis der Materialeigenschaften des Trägers lässt sich somit eine genaue Größe einer auftretenden Kraft bestimmen. Beim Bereitstellen von beispielsweise drei Sensoren, die auf einer gleichen Seite der Okkusionsregion angeordnet sind, entspricht jede Verteilung der gemessenen Intensitäten einem Punkt auf einer eindimensionalen Kurve innerhalb der Okklusionsregion des Trägers. Ein Amplitudenspektrum von den drei Sensoren gibt somit einen Rückschluss auf die entsprechende Position eines Bruxismusereignisses entlang dieser Kurve.

Eine genauere Lokalisierung eines auftretenden Druckes kann durch die Bereitstellung mehrerer Sensoren innerhalb des Trägers und außerhalb der Okklusionsregion des Trägers erreicht werden, indem die Sensoren um die Okklusionsregion herum bzw. an verschiedenen ihrer Seiten angeordnet sind. Dann entspricht jede Verteilung der Amplituden, also der Intensitäten der erfassten Kräfte einem Punkt nicht auf einer eindimensionalen Kurve, sondern auf einer zweidimensionalen Ebene in der Okklusionsregion. Beim Betrachten der Intensitäten aller Sensoren gleichzeitig kann die Intensitätsverteilung bzw. das Intensitätsspektrum der Sensoren als Signatur für einen bestimmten Punkt verstanden werden.

Entsprechend der obigen Darstellungen kann neben dem Vergleich der Intensitäten oder Amplituden der gemessenen Kräfte in der Okklusionsregion des Trägers durch die Sensoren auch ein Vergleich der Laufzeitunterschiede zwischen den Sensoren zum Lokalisieren von okklusalen Kräften durchgeführt werden. Die Laufzeit eines weit entfernten Bruxismusereignisses zu einem Sensor ist länger, als die Laufzeit eines nahen Bruxismusereignisses. Das Bereitstellen mehrerer Sensoren erlaubt damit einen Vergleich zwischen den Laufzeitunterschieden für die Erfassung der Messgrößen durch die Sensoren, um eine okkusale Kraft zu lokalisieren und ihre Größe zu bestimmen.

Bei diesen beschriebenen Verfahren des Lokalisierens und quantitativen Bestimmens von Okklusalen Kräften können mehrere gleichzeitig auftretende okklusale Kräfte an mehreren Okklusionspunkten in der Okklusionsregion des Trägers ebenfalls bestimmt werden. Dies kann erfindungsgemäß dadurch erfolgen, dass sowohl ein Amplitudenvergleich als auch ein Vergleich der Laufzeitunterschiede erfolgt, um somit zusätzliche Parameter für die Ermittlung von Lage und Größe von okklusalen Kräften bereitzustellen.

Diese und weitere Merkmale und Vorteile der Erfindung werden im Folgenden unter Bezugnahme auf die Zeichnungen in beispielhaften Ausführungsformen näher erläutert. Es zeigen:
- Fig. 1A und 1B: schematische Skizzen einer erfindungsgemäßen Vorrichtung in einer ersten Ausführungsform der Erfindung
- Fig. 1C bis 1F: schematische Skizzen von Okklusionspunkten, Okklusionsbereichen und Kauflächen von Zähnen
- Fig. 2A: eine schematische Skizze einer erfindungsgemäßen Vorrichtung in einer weiteren Ausführungsform der Erfindung
- Fig. 2B: eine schematische Skizze einer erfindungsgemäßen Vorrichtung in einer weiteren Ausführungsform der Erfindung
- Fig. 2C: eine schematische Skizze einer erfindungsgemäßen Vorrichtung in einer weiteren Ausführungsform der Erfindung
- Fig. 2D: eine schematische Skizze einer erfindungsgemäßen Vorrichtung in einer weiteren Ausführungsform der Erfindung
- Fig. 3: eine schematische Skizze einer erfindungsgemäßen Vorrichtung in einer weiteren Ausführungsform der Erfindung
- Fig. 4: eine schematische Skizze einer erfindungsgemäßen Vorrichtung in einer weiteren Ausführungsform der Erfindung
- Fig. 5A: eine schematische Skizze einer erfindungsgemäßen Vorrichtung in einer weiteren Ausführungsform der Erfindung
- Fig. 5B: ein Intensitätsdiagramm von Sensoren der Ausführungsform der Erfindung aus Fig. 5A
- Fig. 5C: eine schematische Skizze einer erfindungsgemäßen Vorrichtung in einer weiteren Ausführungsform der Erfindung

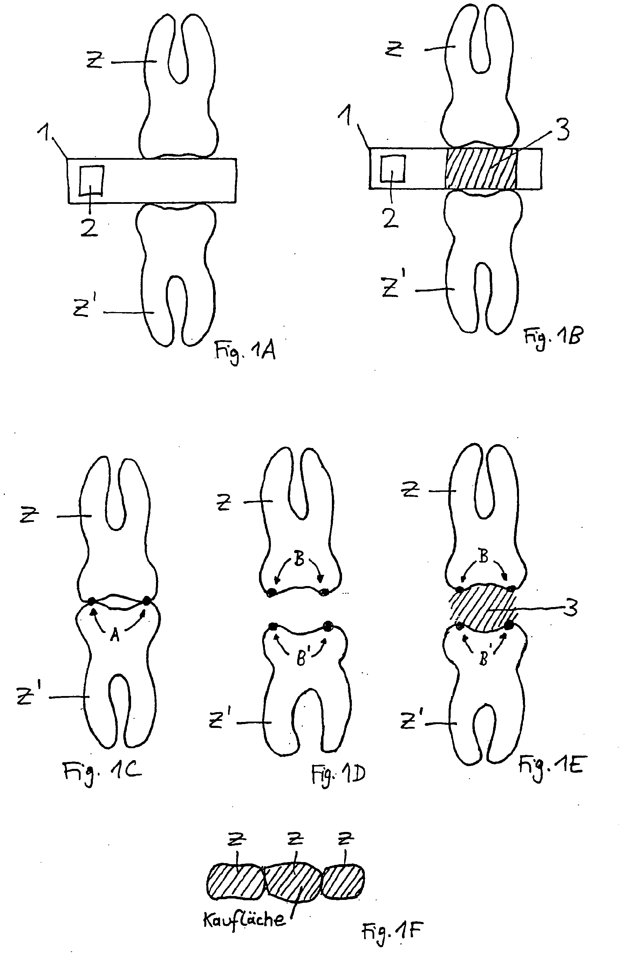

Fig. 1A zeigt eine schematische Skizze einer erfindungsgemäßen Vorrichtung 1 zum Messen okklusaler Kräfte bei einem Schlussbiss von Zähnen mit einem Sensor 2. Es ist die Position von jeweils einem Zahn Z, Z' des Oberkiefers und des Unterkiefers bei einem Schlussbiss auf die Vorrichtung 1 dargestellt.

Fig. 1B zeigt die Vorrichtung 1 nach Fig. 1A und verdeutlicht darüber hinaus, dass der Sensor 2 außerhalb eines Okklusionsbereiches 3 der Zähne Z, Z' angeordnet ist. Zum besseren Verständnis des Okklusionsbereiches 3 wird auf die folgenden Figuren verwiesen, insbesondere auf Fig. 1E.

Zur Veranschaulichung des Begriffes Okklusionsbereich zeigt Fig. 1C zwei antagonistische Zähne Z, Z' bei einem Schlussbiss. Die Punkte A stellen hierbei die Okklusionspunkte der Zähne Z, Z' dar, also jener Punkte, an denen sich der obere und der untere Zahn Z, Z' bei einem Schlussbiss berühren. Es wird von Okklusionspunkten gesprochen, obgleich der Bereich des Antagonistenkontaktes selbstverständlich auch die Form einer Fläche besitzen kann. Im Folgenden wird in Übereinstimmung mit der gängigen Nomenklatur dennoch von Okklusionspunkten gesprochen, es seien jedoch auch ausgedehnte Berührungsstellen der Zähne darunter verstanden.

Fig. 1D zeigt zwei antagonistische Zähne mit den beispielhaften Okklusionspunkten B des oberen Zahnes Z und den Okklusionspunkten B' des unteren Zahnes Z'. Obgleich in der dargestellten Zahnstellung keine Okklusion vorliegt, werden die Punkte B, B' als Okklusionspunkte bezeichnet.

Fig. 1E zeigt die beiden Zähne aus Fig. 1D mit den Okklusionspunkten B, B' der Zähne Z, Z'. Zudem ist ein Okklusionsbereich 3 gezeigt, der jener Bereich zwischen den oberen und unteren Zähnen Z ist, der von den Okklusionspunkten B, B' eingeschlossen wird, also zwischen den Okklusionspunkten B, B' liegt. Nach dem allgemeinen Sprachgebrauch kann der Okklusionsbereich 3 auch als der Bereich beschrieben werden, der zwischen den Kauflächen der oberen und unteren Zähne Z, Z' liegt.

Zur Veranschaulichung zeigt Fig. 1F in der Draufsicht die Kaufläche von drei nebeneinander liegenden Zähnen Z einer Zahnreihe.

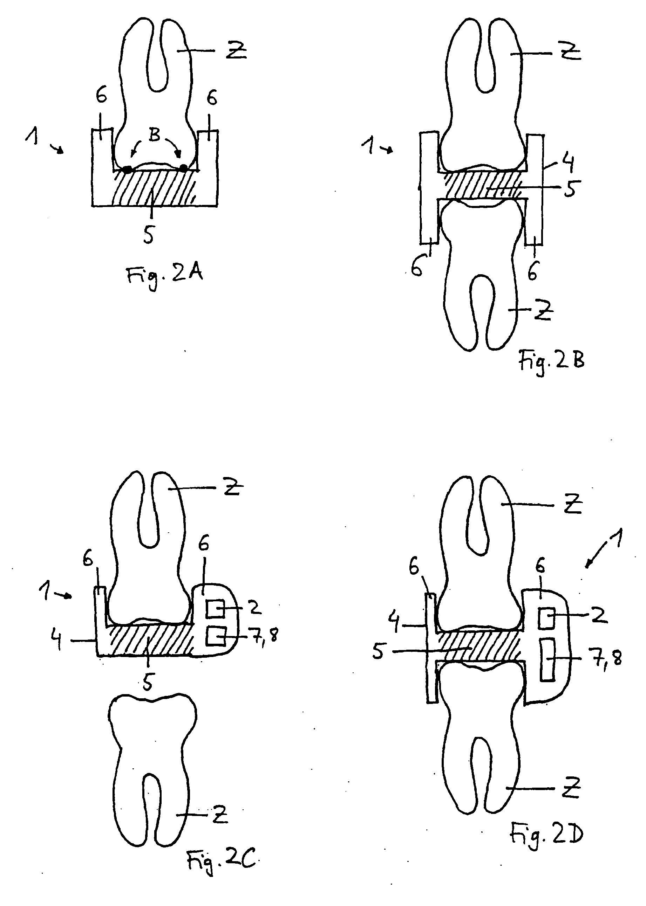

Fig. 2A zeigt schematisch eine Ausführungsform der vorliegenden Erfindung, bei der die Vorrichtung 1 einen Träger 4 umfasst. Der Träger 4 weist eine Okklusionsregion 5 (schraffiert) auf, die, wie gezeigt, sowohl den Bereich des Trägers 4 umfasst, der an Okklusionspunkten B' bzw. der Kaufläche eines Zahnes Z angrenzt, als auch den Teil des Trägers 4 in einem senkrechten Projektionsbereich der Kaufläche. Anders dargestellt, wird die Okklusionsregion 5 des Trägers 4 definiert als der Bereich des Träger 4, der oberhalb oder unterhalb der Kaufläche eines Zahnes Z angeordnet ist. Die Okklusionsregion 5 des Trägers 4 ist somit der Teil des Trägers 4, der im Wesentlichen parallel zur Kaufläche eines Zahnes Z angeordnet ist. Zusätzlich zu einer Okklusionsregion 5 weist der Träger 4 Flankenregionen 6 auf, die im Wesentlichen senkrecht zu der Kaufläche des Zahnes Z angeordnet sind. Eine Flankenregion 6 liegt somit außerhalb einer Okklusionsregion 5 und damit auch außerhalb eines Okklusionsbereiches der Zähne Z. Durch das Bereitstellen einer Okkusionsregion 5 und Flankenregionen 6 des Trägers 4 kann die Vorrichtung 1, wie gezeigt, auf einen Zahn Z aufgeklemmt werden.

Fig. 2B zeigt eine schematische Darstellung einer weiteren Ausführungsform, ähnlich wie in Fig. 2A, mit einem Träger 4, der einen Okklusionsbereich 5 sowie Flankenbereiche 6 aufweist. Die Flankenbereiche 6 sind bei dieser Ausführungsform so geformt, dass sie seitlich nicht nur eines Zahnes Z angeordnet sind, sondern seitlich eines oberen Zahnes Z und eines unteren Zahnes Z'. Somit kann die Vorrichtung 1, wie gezeigt, auf einen Zahn Z der oberen Zahnreihe und auf einen Zahn Z' der unteren Zahnreihe zugleich aufgeklemmt werden.

Fig. 2C zeigt schematisch die Anordnung eines Sensors 2 in einer Vorrichtung 1 gemäß der vorliegenden Erfindung. Der Sensor 2 ist in einer Flankenregion 6 des Trägers 4 und außerhalb einer Okklusionsregion 5 des Trägers angeordnet. Überdies zeigt Fig. 2C die Anordnung einer elektronischen Einheit 7 sowie einer Sendeeinheit 8 in einer Flankenregion 6 des Trägers 4. Die Flankenregionen 6 des Trägers 4 sind so geformt, dass die Anordnung auf mindestens einen Zahn Z der oberen Zähne, oder auf mindestens einen Zahn Z' der unteren Zahnreihe aufklemmbar ist. In dem dargestellten Fall ist die Vorrichtung 1 an mindestens einem Zahn Z des Oberkiefers befestigt.

Fig. 2D zeigt schematisch die Anordnung eines Sensors 2 in einer Vorrichtung 1 gemäß der vorliegenden Erfindung. Der Sensor 2 ist in einer Flankenregion 6 des Trägers 4 und außerhalb einer Okklusionsregion 5 des Trägers angeordnet. Überdies zeigt Fig. 2D die Anordnung einer elektronischen Einheit 7 sowie einer Sendeeinheit 8 in einer Flankenregion 6 des Trägers 4. Bei dieser Ausführungsform sind die Flankenregionen 6 des Trägers 4 so geformt, dass die Anordnung auf mindestens einen Zahn Z der oberen Zähne und auf mindestens einen Zahn Z' der unteren Zahnreihe aufklemmbar ist.

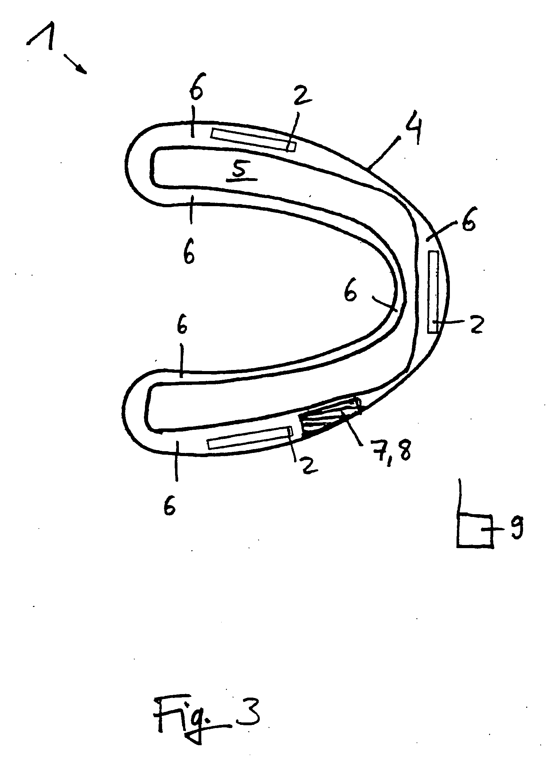

Eine Skizze einer entsprechenden Ausführungsform der Vorrichtung 1 ist in Fig. 3 gezeigt. Drei Sensoren 2 sind innerhalb des Trägers 4, jedoch außerhalb einer Okklusionsregion 5 des Trägers 4 angeordnet. Wie gezeigt, sind die drei Sensoren in einer Flankenregion 6 des Trägers 4 angeordnet. In einer Flankenregion 6 des Trägers 4 ist auch eine elektronische Einheit 7 angeordnet, die von einem Sensor erfasste Messdaten aufnehmen oder auswerten kann. Ebenfalls in einer Flankenregion 6 des Trägers 4 ist eine Sendeeinheit 8 angeordnet, die Messgrößen, die von einem Sensor 2 erfasst wurden oder erfasst werden oder die von der elektronischen Einheit 7 aufgenommen oder ausgewertet wurden oder werden an eine Empfangseinheit 9 senden kann. Wie gezeigt, ist die Empfangseinheit 9 außerhalb der Vorrichtung 1 angeordnet. Sie dient zum Empfang der Messdaten und kann diese an eine weitere Einheit weiterleiten oder durch eine implementierte Signalverarbeitung aufbereiten, weiterverarbeiten oder auswerten.

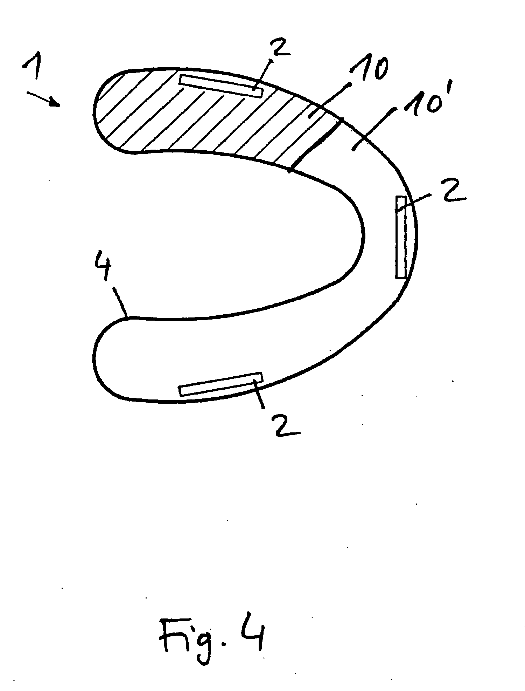

Fig. 4 zeigt eine schematische Skizze einer weiteren Ausführungsform der Erfindung, in welcher drei Sensoren 2 in dem Träger 4 der Vorrichtung 1 angeordnet sind. Der Träger 4 besteht aus zwei Bereichen 10, 10' mit unterschiedlichen mechanischen Eigenschaften, wie Härte oder Steifigkeit. Ein Sensor 2 ist innerhalb des einen Bereiches 10 angeordnet, während zwei Sensoren 2 innerhalb des anderen Bereiches 10' angeordnet sind. Auf diese Weise kann eine Datenverarbeitung der Messwerte der Sensoren bei Kenntnis der entsprechenden mechanischen Koeffizienten bzw. Eigenschaften der unterschiedlichen Bereiche 10, 10' um Parameter erweitert werden.

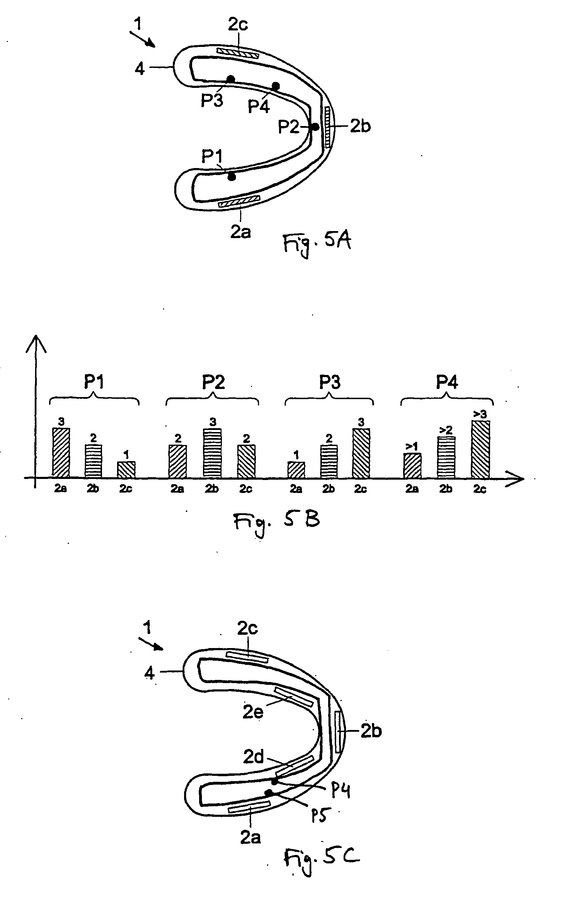

Fig. 5A zeigt eine schematische Skizze einer Vorrichtung 1 zum Messen okklusaler Kräfte bei einem Schlussbiss von Zähnen mit drei Sensoren 2a, 2b, 2c, die innerhalb des Trägers 4 der Vorrichtung 1 und außerhalb der Okklusionsregion 5 angeordnet sind. Durch die Anordnung der Sensoren 2a, 2b, 2c entlang der Okklusionsregion 5 kann ein Bruxismusereignis, also beispielsweise ein Druck auf einen Punkt P1, P2, P3 oder P4 in der Okklusionsregion 5, entlang einer eindimensionalen Kurve lokalisiert werden. Dazu können die an den Sensoren 2a, 2b, 2c gemessenen Druckintensitäten verglichen werden, wie sie in Fig. 5B beispielhaft dargestellt sind. Jede Verteilung der Intensitäten 1, 2, 3 (Ordinate), die an den Sensoren 2a, 2b, 2c gemessen werden, entspricht einem bestimmten Punkt entlang einer Linie in der Okklusionsregion 5. Die ersten drei Werte 3, 2, 1 für die Intensitäten der Sensoren 2a, 2b, 2c geben zusammen betrachtet eine Intensitäts-Signatur für einen Punkt P1 entsprechend Fig. 5A wieder. Dementsprechend geben die zweiten, dritten und vierten Wertetripel 2, 3, 2 und 1, 2, 3 und >1, >2, >3 der Intensitäten der Sensoren 2a, 2b, 2c zusammen betrachtet Intensitäts-Signaturen für die Punkte P2, P3 und P4 aus Fig. 5A wieder.

Fig. 5C zeigt eine schematische Skizze einer Vorrichtung 1 zum Messen okklusaler Kräfte nach einer weiteren Ausführungsform der Erfindung. Innerhalb des Trägers 4 der Vorrichtung 1 und außerhalb der Okklusionsregion 5 des Trägers 4 sind fünf Sensoren 2a bis 2e angeordnet. Durch die Anordnung der Sensoren 2a bis 2e um die Okklusionsregion 5 des Trägers 4 bzw. der Vorrichtung 1 herum kann ein Bruxismusereignis, also beispielsweise ein Druck auf einen Punkt P4, P5 in der Okklusionsregion 5, innerhalb einer zweidimensionalen Fläche lokalisiert werden. Dazu werden die an den Sensoren 2a bis 2e gemessenen Druckintensitäten oder Laufzeitunterschiede der Sensormessungen verglichen.

## Patentansprüche

1. Zahnschiene (1) zum Messen okklusaler Kräfte bei einem Schlussbiss von Zähnen, mit wenigstens einem Sensor (2) und einem Träger (4) mit einer Okklusionsregion (5), die dafür vorgesehen ist, mit einer Kaufläche eines Zahnes In Kontakt zu treten, wobei
- die Okklusionsregion (5) des Trägers (4) Im Wesentlichen parallel zu einer Kaufläche der Zähne anordenbar ist;
- der Träger (4) Flankenregionen (6) aufweist, die im Wesentlichen senkrecht zur Kaufläche der Zähne anordenbar sind;
- der Träger (4) mittel der Okklusionsregion (5) und der Flankenregionen (6) auf mindestens einen Zahn aufklemmbar ist, **dadurch gekennzeichnet, dass**
- der Sensor (2) innerhalb einer der Flankenregionen (6) des Trägers (4). Jedoch außerhalb der Okklusionsregion (5) angeordnet ist, so dass der Sensor (2) vollständig vom Träger (4) umschlossen ist.

2. Zahnschiene (1) nach Anspruch 1, **dadurch gekennzeichnet, dass** der Sensor (2) an der Außenseite eines Zahnes, an der Außenseite einer Zahnfüllung, einer Plombe, einer Krone oder eines Inlays eines Zahnes, oder an der Außenseite eines Zahnimplantates, eines Kunstzahnes oder einer Zahnprothese anordenbar ist.

3. Zahnschiene (1) nach einem der Ansprüche 1 oder 2, **dadurch gekennzeichnet, dass** der Sensor (2) ein Dehnungssensor, ein Biegungssensor, ein Vibrationssensor, ein Spannungssensor, ein Verschiebungssensor, ein Drucksensor oder ein Kraftsensor ist.

4. Zahnschiene (1) nach einem der Ansprüche 1 bis 3, **dadurch gekennzeichnet, dass** der Sensor (2) ein piezoelektrischer Sensor oder ein Dehnungsmessstreifen ist.

5. Zahnschiene (1) nach einem der Ansprüche 1 bis 4, **dadurch gekennzeichnet, dass** mehrere Sensoren (2) mit unterschiedlichem Aufbau oder unterschiedlicher Funktion vorgesehen sind.

6. Zahnschiene (1) nach einem der Ansprüche 1 bis 5, **dadurch gekennzeichnet, dass** eine elektronische Einheit (7) vorgesehen ist, zum Aufnehmen und/oder zum Auswerten von durch den Sensor (2) erfassten Messgrößen.

7. Zahnschiene (1) nach einem der Ansprüche 1 bis 6, **gekennzeichnet durch** eine Sendeeinheit (8) zum Senden von **durch** den Sensor (2) erfassten oder von der elektronischen Einheit (7) aufgenommenen oder ausgewerteten Messgrößen an eine Empfangseinheit (9).

8. Zahnschiene (1) nach einem der Ansprüche 1 bis 7, **dadurch gekennzeichnet, dass** der Träger (4) mindestens zwei unterschiedliche Bereiche (10. 10') mit unterschiedlicher Härte, Steifigkeit oder Festigkeit aufweist.

9. Zahnschiene (1) nach Anspruch 8, **dadurch gekennzeichnet, dass** mindestens zwei Sensoren (2) vollständig innerhalb von verschiedenen der unterschiedlichen Bereiche (10. 10') angeordnet sind.

10. Zahnschiene (1) nach Anspruch 9, **dadurch gekennzeichnet, dass** mindestens zwei Sensoren (2) an verschiedenen der unterschiedlichen Bereiche (10, 10') angrenzend angeordnet sind.

11. System zur Diagnose oder Therapie von Bruxismus, mit einer Zahnschiene (1) nach einem der Ansprüche 1 bis 10 und einer Empfangseinheit (9) zum Empfangen von mit der Sendeeinheit (8) gesendeten Messgrößen.

12. Verfahren zum Bestimmen von Lage oder Größe okklusaler Kräfte auf eine Zahnschiene (1) nach einem der Ansprüche 1 bis 10, mit den Schritten:
- Bereitstellen von mindestens zwei Sensoren (2) durch Anordnen innerhalb des Trägers (4)
- Lokalisieren oder quantitatives Bestimmen von okklusalen Kräften über einen Vergleich von Amplituden oder Laufzeitunterschieden von durch die Sensoren (2) an eine elektronische Einheit (8) bereitgestellten Messgrößen durch die elektronische Einheit (8).

## Claims

1. A dental splint (1) for the measurement of occlusal forces in case of a terminal occlusion of teeth, comprising at least one sensor (2) and a support (4) having an occlusion region (5) which is provided for contacting an occlusal surface of a tooth, wherein
- the occlusion region (5) of the support (4) is, in essence, arrangeable in parallel to an occlusal surface of the teeth;
- the support (4) comprises a flank region (6) which is, in essence, arrangeable perpendicular to the occlusal surface of the teeth;
- the support (4) is clampable onto at least one tooth by the occlusal region (5) and the flank region (6), **characterized in that**
- the sensor (2) is arranged within the flank region (6) of the support (4) but exterior with respect to the occlusal region (5), such that the sensor (2) is completely enclosed by the support (4).

2. A dental splint (1) according to Claim 1, **characterized in that** the sensor (2) is arrangeable on the outside of a tooth, on the outside of a dental filling, a plombage, a crown or an inlay of a tooth, or on the outside of a dental implant, an artificial tooth or a dental prosthesis.

3. A dental splint (1) according to anyone of Claims 1 or 2, **characterized in that** the sensor (2) is an expansion sensor, a bending sensor, a vibration sensor, a tension sensor, a displacement sensor, a pressure sensor, or a force sensor.

4. A dental splint (1) according to anyone of Claims 1 to 3, **characterized in that** the sensor (2) is a piezoelectric sensor or a strain gauge.

5. A dental splint (1) according to anyone of Claims 1 to 4, **characterized in that** a plurality of sensors (2) differing in structure or differing in function are provided.

6. A dental splint (1) according to anyone of Claims 1 to 5, **characterized in that** an electronic unit (7) is provided for taking up and/or evaluating measured variables registered by the sensor (2).

7. A dental splint (1) according to anyone of Claims 1 to 6, **characterized by** a transmitter unit (8) for transmitting measured variables registered by the sensor (2) or taken up or evaluated by the electronic unit (7) to a receiver unit (9).

8. A dental splint (1) according to anyone of Claims 1 to 7, **characterized in that** the support (4) comprises at least two different regions (10, 10') having a different hardness, stiffness or strength.

9. A dental splint (1) according to Claim 8, **characterized in that** at least two sensors (2) are completely arranged within various ones of the different regions (10, 10').

10. A dental splint (1) according to Claim 9, **characterized in that** at least two sensors (2) are arranged adjacent to various ones of the different regions (10, 10').

11. A system for the diagnosis or treatment of bruxism, comprising a dental splint (1) according to anyone of Claims 1 to 10 and a receiver unit (9) for receiving measured variables transmitted by the transmitter unit (8).

12. A method for the determination of the position or size of occlusal forces on a dental splint (1) according to anyone of Claims 1 to 10, comprising the steps of:
- providing at least two sensors (2) by arrangement inside the support (4);
- localizing or quantitatively determining occlusal forces by an electronic unit (8) comparing amplitudes or runtime differences of measured variables which the sensors (2) provide to the electronic unit (8).

## Revendications

1. Gouttière dentaire (1) pour mesurer les forces occlusales, quand on serre les dents, avec au moins un capteur (2) et un support (4) présentant une zone d'occlusion (5) qui est destinée à venir en contact avec une surface de mastication d'une dent, étant précisé que
- la zone d'occlusion (5) du support (4) est apte à être disposée sensiblement parallèlement à une surface de mastication des dents ;
- que le support (4) présente des zones de flanc (6) qui sont aptes à être disposées sensiblement perpendiculairement à la surface de mastication des dents ;
- que le support (4) est apte à être serré sur au moins une dent à l'aide de la zone d'occlusion (5) et des zones de flanc (6),
**caractérisée**
- **en ce que** le capteur (2) est disposé à l'intérieur de l'une des zones de flanc (6) du support (4), mais à l'extérieur de la zone d'occlusion (5), de sorte qu'il est complètement entouré par le support (4).

2. Gouttière dentaire (1) selon la revendication 1, **caractérisée en ce que** le capteur (2) est apte à être disposé sur le côté extérieur d'une dent, sur le côté extérieur d'un amalgame, d'un plombage, d'une couronne ou d'un inlay d'une dent, ou sur le côté extérieur d'un implant dentaire, d'une dent artificielle ou d'une prothèse dentaire.

3. Gouttière dentaire (1) selon l'une des revendications 1 ou 2, **caractérisée en ce que** le capteur (2) est un capteur d'allongement, un capteur de flexion, un capteur de vibrations, un capteur de tension, un capteur de déplacement, un capteur de pression ou un capteur de force.

4. Gouttière dentaire (1) selon l'une des revendications 1 à 3, **caractérisée en ce que** le capteur (2) est un capteur piézoélectrique ou une jauge extensométrique.

5. Gouttière dentaire (1) selon l'une des revendications 1 à 4, **caractérisée en ce qu'**il est prévu plusieurs capteurs (2) avec des structures ou des fonctions différentes.

6. Gouttière dentaire (1) selon l'une des revendications 1 à 5, **caractérisée en ce qu'**il est prévu une unité électronique (7) pour enregistrer et/ou analyser des grandeurs de mesure détectées par le capteur (2).

7. Gouttière dentaire (1) selon l'une des revendications 1 à 6, **caractérisée par** une unité d'émission (8) destinée à envoyer à une unité de réception (9) des grandeurs de mesure détectées par le capteur (2) ou enregistrées ou analysées par l'unité électrique (7).

8. Gouttière dentaire (1) selon l'une des revendications 1 à 7, **caractérisée en ce que** le support (4) présente au moins deux zones différentes (10, 10') avec des duretés, des rigidités ou des solidités différentes.

9. Gouttière dentaire (1) selon la revendication 8, **caractérisée en ce qu'**au moins deux capteurs (2) sont disposés entièrement à l'intérieur de plusieurs zones différentes (10, 10').

10. Gouttière dentaire (1) selon la revendication 9, **caractérisée en ce qu'**au moins deux capteurs (2) sont disposés près de plusieurs zones différentes (10, 10').

11. Système de diagnostic ou de thérapie pour le bruxisme, avec une gouttière (1) selon l'une des revendications 1 à 10, et une unité de réception (9) pour recevoir des grandeurs de mesure envoyées à l'aide de l'unité d'émission (8).

12. Procédé pour définir la position ou le degré de forces occlusales exercées sur une gouttière (1) selon l'une des revendications 1 à 10, avec les étapes :
- de mise en place d'au moins deux capteurs (2) en les disposant à l'intérieur du support (4),
- de localisation ou de définition quantitative, par une unité électronique (8), de forces occlusales grâce à une comparaison d'amplitudes ou de différences de durée de grandeurs de mesure fournies par les capteurs (2) à l'unité électronique (8).
